(19) **Europäisches Patentamt**

**European Patent Office**

**Office européen des brevets**

(11) **EP 1 314 435 A1**

(12) **EUROPÄISCHE PATENTANMELDUNG**

(43) Veröffentlichungstag:
28.05.2003 Patentblatt 2003/22

(51) Int Cl.⁷: **A61K 35/78**, A61P 17/00

(21) Anmeldenummer: 02002526.8

(22) Anmeldetag: 04.02.2002

(84) Benannte Vertragsstaaten:
**AT BE CH CY DE DK ES FI FR GB GR IE IT LI LU MC NL PT SE TR**
Benannte Erstreckungsstaaten:
**AL LT LV MK RO SI**

(30) Priorität: **27.11.2001 CH 21832001**

(71) Anmelder: **RAHN AG**
**8050 ZÜRICH (CH)**

(72) Erfinder: **Haltinner, Marcel**
**CH-8500 Frauenfeld (CH)**

(74) Vertreter: **Spierenburg, Pieter**
**Spierenburg & Partner AG**
**Patent- und Markenanwälte**
**Mellingerstrasse 12**
**5443 Niederrohrdorf (CH)**

(54) **Kosmetische und/oder dermatologische Wirkstoffzubereitung**

(57) Es wird eine neue kosmetische und/oder dermatologische Wirkstoffzubereitung, vorgestellt, die folgende Bestandteile aufweist: (a) einen durch Extraktion hergestellten Pflanzenextrakt, insbesondere Melissenextrakt, der in angereicherter Form Rosmarinsäure enthält, (b) einen durch Extraktion hergestellten Getreideextrakt, insbesondere Gerstenextrakt, der die Spurenelemente Selen, Zink und Mangan enthält, und (c) einen aus der Gruppe der Flavonoide ausgewählten Antioxidativum. Diese neue Wirkstoffzubereitung hat den grossen Vorteil, dass sie in einem kosmetisch und/oder dermatologisch geeigneten Vehikel einen synergistisch wesentlich erhöhten Radikalschutzfaktor aufweist.

EP 1 314 435 A1

**Beschreibung**

Die Erfindung betrifft eine kosmetische und/oder dermatologische Wirkstoffzubereitung nach dem Oberbegriff des Patentanspruchs 1.

Die erfindungsgemässe kosmetische und/oder dermatologische Wirkstoffzubereitung als Bestandteil kosmetische und/oder dermatologischer Präparate schützt in besonders wirksamer Weise gegen den Angriff freier Radikale auf die Haut sowohl allein als auch in Kombination mit anderen Wirkstoffen.

Bekanntlich sind freie Radikale, wie Superoxid-Ionen, Hydroxy-Radikale, Oxide usw. für einen Gewebeabbau und damit für die Erscheinung des Alterns der Haut verantwortlich. Die Proteine und Lipide der Zellmembranen werden zerstört, die DNA beschädigt und eine Schlüsselsubstanz der Haut, Hyaluronsäure, wird ebenfalls abgebaut. Unter normalen biologischen Bedingungen gibt es ein Gleichgewichtsverhältnis zwischen auftretenden freien Radikalen und deren Eindämmung durch körpereigene chemische oder enzymatische Systeme. Durch zusätzliche Stressfaktoren von aussen, wie aggressive Atmosphäre, Tabakrauch, Ultraviolettstrahlung usw. werden diese körpereigenen Abwehrsysteme überfordert und das Gleichgewicht zugunsten der freien Radikale gestört, und ein Ausgleich durch topisch applizierte Produkte angezeigt ist. Es treten Entzündungs- oder Alterungserscheinungen der Haut auf, und ein Ausgleich durch kosmetische und/oder dermatologische Produkte ist angezeigt.

Es sind bereits eine Reihe von Produkten für diesen Zweck vorgeschlagen worden, die oftmals Vitaminmischungen mit den Vitaminen A, C und E sowie Polyphenolen enthalten oder Zusätze von Superoxiddismutase oder Extrakte aus bestimmten pflanzlichen oder tierischen Lebewesen. So ist beispielsweise aus US-A-5,629,185 eine kosmetische Zusammensetzung bekannt, die Ultraschallaufschlussprodukte von Hefen oder anderen Zelldispersionen enthält. Weiterhin gibt es eine Vielzahl von Veröffentlichungen, in denen die Verwendung reiner Pflanzenextrakte für kosmetische Zwecke beschrieben ist, wie z.B. in der WO97/45100 mit einem Gemisch von sieben verschiedenen Extrakten zur Bekämpfung von Cellulite.

Die Suche nach weiteren wirksamen Stoffen ist ein wesentliches Element kosmetischer und dermatologischer Forschung. Weiterhin problematisch bei vielen dieser Produkte ist es, dass die gegen freie Radikale wirksamen Stoffe ihr Fängerpotential innerhalb der fertigen kosmetischen Zusammensetzung oftmals nicht auf Dauer beibehalten, z.B. Polyphenole und Polycyanooligomere, welche stark nachdunkeln. Reines Vitamin C ist als guter Radikalfänger bekannt, aber in der galenischen Formuierung schwer zu stabilisieren. Deshalb werden stabilere Vitamin-C Derivate in Form von Palminate und Phosphate verwendet, was jedoch die Eigenschaft als Radikalfänger wesentlich limitiert.

Der vorliegenden Erfindung liegt nun die Aufgabe zugrunde, eine Wirkstoffzubereitung anzugeben, bei der das Schutzpotential gegen Radikale wesentlich erhöht wird und hauptsächlich auf pflanzlicher Basis hergestellt ist.

Diese Aufgabe wird erfindungsgemäss durch eine kosmetische und/oder dermatologische Wirkstoffzubereitung nach den Merkmalen des Patentanspruchs 1 gelöst.

Die erfindungsgemässe Wirkstoffzubereitung hat den grossen Vorteil, dass sie in einem kosmetisch oder dermatologisch geeigneten Träger einen synergistisch wesentlich erhöhten Radikalschutzfaktor aufweist. Gemäss Anspruch 1 weist sie folgende Bestandteile auf: (a) einen durch Extraktion hergestellten Melisseextrakt, der in aufkonzentrierter Form Rosmarinsäure enthält, (b) einen durch Extraktion hergestellten Gerstenextrakt, der die Spurenelemente Selen, Zink und Mangan in standardisierten Mengen enthält, und (c) einen aus der Gruppe der Flavonoide ausgewählten Antioxidativum. Die vorliegende Erfindung zeigt gerade in Punkto Stabilität grosse Vorteile gegenüber den bekannten Stoffen, ohne negativen Einfluss auf die Wirksamkeit als Radikalfänger. Vorzugsweise ist das Planzenextrakt ein Melissenextrakt gemäss Anspruch 2. Als bevorzugtes Antioxidativum wird gemäss Anspruch 3 Disodiumrutinyldisulfat gewählt. Es hat sich als besonders vorteilhaft erwiesen, wenn der Anteil der Bestandteile nach Anspruch 4 in folgenden Bereichen liegt: Pflanzenextrakt gemäss (a) 1-5 Gew.-%; Gerstenextrakt gemäss (b) 5-10 Gew.-%; Antioxidativum aus der Gruppe der Flavonoide gemäss (c) 1-5 Gew.-%. Gemäss Anspruch 5 enthält der Extrakt (a) in der Praxis 10 bis 15 Gew.-%, vorzugsweise etwa 12 Gew.-%, Rosmarinsäure. Vorteilhafterweise nach Anspruch 6 weist die Zubereitung einen Radikalschutzfaktor im Bereich von 1,47 bis 7,15 gemäss in-vivo Messung auf. Gemäss Anspruch 7 kann die Wirkstoffzubereitung weiterhin eine oder mehrere der folgenden Bestandteile enthalten: (1) Extrakte oder behandelte Extrakte freie Radikale bindenden Pflanzen, (2) Vitamine A, C und E, (3) synthetisch hergestellte Antioxidansien, (4) übliche Wirkstoffe, Hilfsstoffe und Trägerstoffe wie Urea, Emulgatoren, Hydrocolloiden. Gemäss Anspruch 8 findet die erfindungsgemässe Wirkstoffzubereitung mit grossen Vorteil ihre Anwendung in der Pflege von belasteter und/oder geschädigter Haut. Die Verwendung der und/oder dermatologischen Wirkstoffzubereitung hat sich gemäss Anspruch 9 besonders bewährt bei der Herstellung einer Wirkstoffzubereitung eines kosmetischen und/oder dermatologischen Vehikels zur Behandlung von belasteter und/oder geschädigter Haut.

Weitere Vorteile der Erfindung folgen aus den abhängigen Patentansprüchen und aus der nachfolgenden Beschreibung, in welcher die Erfindung anhand eines Ausführungsbeispieles näher erläutert wird.

Die erfindungsgemässe Zubereitung des neuen Wirkstoffes besteht darin, dass aus einer Zusammensetzung von 5 bis 10 Gew.-% flüssigen Gerstenextrakt, 1 bis 5 Gew.-% Pflanzenextrakt, insbesondere Melissenextrakt, und 1 bis 5 Gew.-% Disodiumrutinyldisulfat in einem Mischer während einer Zeitspanne von 30 bis 60 Minuten bei einer Temperatur von etwa 25 °C gründlich gerührt wird. Dadurch entsteht eine klare, gelb-bräunliche wässrige Lösung, die dank ihrer dezenten Farben ideal in kosmetische und/oder dermatologische Produkte eingearbeitet werden kann.

Melissenextrakt wird in einem Wasserbad von etwa 80 °C aus Melissenblätter gewonnen. Die meisten Bestandteile des Melissenöls sind lipophil und finden sich daher im hergestellten wässrigen Extrakt nur in Spuren. Weitere Einzelheiten dazu können dem Artikel von H. Eggensperger et. al. in SÖFW-Journal, 126. Jg. 1/2-2000, Seiten 25 bis 29 entnommen werden. Unter schonender Destillation und Vakuumtrocknung bei einer Temperatur unter 55 °C wird nun der wässerige Melissenextrakt einkonzentriert. Mit Ethanol wird der Trockenextrakt nochmals aufgelöst und die Balaststoffe entfernt. Der Ethanol wird unter sehr schonenden Konditionen mittels Destillation und Vakuumtrocknung entfernt. Durch dieses Herstellverfahren kann ein Trockenextrakt hergestellt werden, der in der Farbe sehr gut in ein kosmetisches und/oder dermatologisches Produkt eingearbeitet werden kann, d.h. nicht dunkel ist, und ein Rosmarinsäuregehalt von über 12 Gew.-% aufweist. Die Rosmarinsäure ist bekannt als eines der besten Antioxidanten (vgl. die Artikel von H. Eggensperger et. al. in SÖFW-Journal, 124. Jg., 10/98, Seiten 563 bis 567 und Seiten 634 bis 642).

Gerstenextrakt wird durch ein an sich bekanntes Extraktionsverfahren aus Gerste hergestellt. Es ist bekannt, dass Gerste reich an Selen, Zink und Mangan ist. Der wässrige Gerstenextrakt ist standardisiert in Selen, Zink und Mangan. Diese Spurenelemente sind dafür bekannt, dass sie das hauteigene Schutzsystem zur Nachweisung freier Radikale katalytisch unterstützen. Durch das Vorhandensein dieser Elemente werden die Reaktionen schneller und effizienter zu Ende geführt. Mangan, Zink und Selen wirken als Katalysatoren zum reibungslosen Ablauf des Prozesses. Anstelle von Gerstenextrakt kann auch ein anderes Getreideextrakt, wie z.B. aus Weizen, Hafer oder Roggen, verwendet werden.

Der weitere Bestandteil, Disodiumrutinyldisulfat, ist ein Derivat von Rutin, das zu den Flavonoiden gehört und starke antioxidative Eigenschaften hat (siehe Merkblatt "Rutin & Quercitin" der Firma Merck KGaA, D-64271 Darmstadt). Disodiumrutinyldisulfat ist wasserlöslich, im Gegensatz zu Rutin selber. Rutin wirkt gegen freie Radikale, d.h. gegen die Hautalterung und hemmt die Lipidperoxidation, was ebenfalls ein Alterungsprozess ist.

Die nun derart hergestellte Hautschutzemulsion mit einer 5-%igen Wirkstoffzubereitung wurde von einem unabhängigen Testinstitut in einem Testverfahren an 10 verschiedenen Testpersonen erprobt. Dabei wurde diese Emulsion, Produkt A, und ein entsprechendes Placebo-Produkt, Produkt B, auf den Rücken der Testpersonen angebracht. Das Placebo-Produkt hat dabei aus der Tabelle 1 folgenden Zusammensetzung:

Tabelle 1

| Phase | Rohstoff | INCI-Name | % [w/w] |
|---|---|---|---|
| A | Wasser demin. | Aqua | 86.60 |
| | Glycerin 86% | Glycerin | 3.00 |
| | Carbopol ETD 2020 | Acrylates / C 10-30 Akryl Acrylate Crosspolymer | 0.40 |
| | NaOH-Lösung 10% | Sodium Hydroxide, Aqua | 1.20 |
| B | Cetiol SN | Cetearyl Isononanoate | 2.50 |
| | Cetiol OE | Dicaprylyl Ether | 5.00 |
| | Uniphen | Phenoxyethanol, Methylparaben | 1.00 |
| | Parfum | Ethylparaben, Propylparaben, Butylparaben Parfum | 0.10 |
| C | Keltrol F | Xanthan Gum | 0.20 |
| | | | 100.00 |

Das untersuchte Material wird durch Abstreifen der Hautoberfläche analysiert. Die freien Radikale wurden nach Einfärbung und mit Hilfe eines Fluoroscans sichtbar gemacht und quantitativ ausgewertet. Die Menge bei der Abstreifung erhaltenen Zellen des Stratum Corneum wurden mittels einer Bildanalyse ermittelt. Die kutanen Peroxide wurden in einer Fluoreszenzprobe beobachtet. Die UV-Bestrahlung mit UVA und UVB wurde in standardmässigen Dosen durchgeführt. Der Anteil an kutanen Peroxiden in den Abstreifungen wurden per $cm^2$ Zellen berechnet.

Der Testversuch wurde wie folgt durchgeführt:

Am ersten Tag wurden vier kutane Zonen auf dem Rücken der 10 Testpersonen einheitlich festgelegt. Dabei wurde unterteilt in vier Zonen: (a) eine nicht-bestrahlte, nicht-behandelte Zone, (b) eine bestrahlte, nicht-behandelte Zone, (c) eine bestrahlte Zone, behandelt mit Produkt A, (d) eine bestrahlte Zone, behandelt mit Produkt B. Dabei wurden die Zonen (c) und (d) mit einer Menge von 2µl/$cm^2$ jede zweite Stunde während sechs Stunden mit den Produkten behandelt.

Am zweiten Tag wurden die aufeinanderfolgenden Abstreifungen in jeder Zone durchgeführt und die Menge der Zellen gemessen. Die Dosis der Peroxide wurde mittels Farbbestimmung mit Fluoreszenz ermittelt.

Das Resultat der Untersuchung ist in der Tabelle 2 dargestellt. Sie zeigt die individuellen Werte und die Mit-

telwerte der Fluoreszenz pro cm$^2$ (UF/cm$^2$), den Standardfehler des Mittelwertes (SFM), das Minimum, das Maximum und die Mediane aus dem Student's Test, die für die Testpersonen mit dem Produkt A und dem Placebo-Produkt B erhalten wurden. Die Wirkung gegen freie Radikale (E %) kann durch folgende Formel berechnet werden:

$$E \% = (ZI - ZIT) / (ZI - ZNINT) \times 100 \tag{1}$$

wobei:

ZI          die bestrahlte, nicht-behandelte Zone,
ZNINT    die nicht-bestrahlte, nicht-behandelte Zone,
ZIT         die bestrahlte Zone, behandelt mit Produkt A oder B

Der Radikalschutzfaktor wurde dabei nach einer gängigen in-vivo Messmethode bestimmt.

Tabelle 2

| Testperson Nr. | ZNINT | ZI | ZIT / Produkt A | ZIT / Produkt B |
|---|---|---|---|---|
| 1 | 4,81 | 6,94 | 1,93 | 4,27 |
| 2 | 1,47 | 1,96 | 0,22 | 1,94 |
| 3 | 1,89 | 2,37 | 0,17 | 3,08 |
| 4 | 7,22 | 9,14 | 1,75 | 6,15 |
| 5 | 8,07 | 9,25 | 1,74 | 6,90 |
| 6 | 3,16 | 5,06 | 1,30 | 3,91 |
| 7 | 3,48 | 4,82 | 1,92 | 6,99 |
| 8 | 1,34 | 2,52 | 1,60 | 2,87 |
| 9 | 3,30 | 4,17 | 3,92 | 7,15 |
| 10 | 2,45 | 4,15 | 1,11 | 1,47 |
| **Mittelwert** | **3,52** | **4,77** | **1,51** | **4,26** |
| Median | 3,16 | 4,17 | 1,60 | 3,91 |
| Minimum | 1,34 | 1,96 | 0,17 | 1,47 |
| Maximum | 8,07 | 9,25 | 3,92 | 7,15 |
| SFM | 0,69 | 0,80 | 0,31 | 0,66 |
| E % | | | 261 % | 41 % |
| Student's Test | | | 0,001 | 0,43 |

Aus der Untersuchung kann man die Schlussfolgerung ziehen, dass das Produkt A die kutanen Peroxide beträchtlich verringert (E = 261 %) und somit eine sehr gute Wirkung gegen Radikale aufweist. Das Placebo-Produkt B zeigt eine geringe Wirkung gegen Radikale (E = 41 %).

**Patentansprüche**

1.    Kosmetische und/oder dermatologische Wirkstoffzubereitung enthaltend:

      (a) einen durch Extraktion hergestellten Pflanzenextrakt, der in angereicherter Form Rosmarinsäure enthält,
      (b) einen durch Extraktion hergestellten Getreideextrakt, insbesondere Gerstenextrakt, der die Spurenelemente Selen, Zink und Mangan enthält, und
      c) einen aus der Gruppe der Flavonoide ausgewählten Antioxidativum.

2.    Zubereitung nach Anspruch 1, **dadurch gekennzeichnet, dass** das Pflanzenextrakt Melissenextrakt ist.

**3.** Zubereitung nach Anspruch 1 oder 2, **dadurch gekennzeichnet, dass** das Antioxidativum Disodiumrutinyldisulfat ist.

**4.** Zubereitung nach einem der Ansprüche 1 bis 3, **dadurch gekennzeichnet, dass** der Anteil der Bestandteile in folgenden Bereichen liegt: Pflanzenextrakt gemäss (a) 1-5 Gew.-%; Gerstenextrakt gemäss (b) 5-10 Gew.-%; Antioxidativum aus der Gruppe der Flavonoide gemäss (c) 1-5 Gew.-%.

**5.** Zubereitung nach einem der Ansprüche 1 bis 4, **dadurch gekennzeichnet, dass** der Extrakt (a) 10 bis 15 Gew.-%, vorzugsweise etwa 12 Gew.-%, Rosmarinsäure enthält.

**6.** Zubereitung nach einem der Ansprüche 1 bis 5, **gekennzeichnet durch** einen Radikalschutzfaktor im Bereich von 1,47 bis 7,15 gemäss in-vivo Messung.

**7.** Zubereitung nach einem der Ansprüche 1 bis 6, **dadurch gekennzeichnet, dass** die Wirkstoffzubereitung weiterhin eine oder mehrere der folgenden Bestandteile enthält: (1) Extrakte oder behandelte Extrakte von freie Radikale bindenden Pflanzen, (2) Vitamine A, C und E, (3) synthetisch hergestellte Antioxidansien, und (4) übliche Wirkstoffe, Hilfsstoffe und Trägerstoffe wie Urea, Emulgatoren, Hydrocolloiden.

**8.** Kosmetische und/oder dermatologische Wirkstoffzubereitung nach einem der Ansprüche 1 bis 7 zur Anwendung in der Therapie von belasteter und/oder beschädigter Haut.

**9.** Verwendung der kosmetischen und/oder dermatologischen Wirkstoffzubereitung nach einem der Ansprüche 1 bis 7 zur Herstellung einer Wirkstoffzubereitung mit einem kosmetischen und/oder dermatologischen Vehikels zur Behandlung von belasteter und/oder beschädigter Haut.

**Europäisches Patentamt**

## EUROPÄISCHER RECHERCHENBERICHT

Nummer der Anmeldung

EP 02 00 2526

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| X | EDWIN N. FRANKEL ET AL.: "Antioxidant activity of a rosemary extract and its constituents, carnosic acid, carnosol, and rosmarinic acid, in bulk oil and oi-in-water emulsion" JOURNAL OF AGRICULTURAL AND FOOD CHEMISTRY, Bd. 44, Nr. 1, 1996, Seiten 131-135, XP000546463 * das ganze Dokument * --- | 1,7 | A61K35/78 A61P17/00 |
| X | YANISHLIEVA NEDYALKA V ET AL: "Stabilisation of edible oils with natural antioxidants." EUROPEAN JOURNAL OF LIPID SCIENCE AND TECHNOLOGY, Bd. 103, Nr. 11, November 2001 (2001-11), Seiten 752-767, XP001080680 ISSN: 1438-7697 * Seite 754, linke Spalte * --- | 1,7 | |
| Y | SYLVESTER N. ONYENEHO ET AL.: "Antioxidant activity of durum wheat bran" JOURNAL OF AGRICULTURAL AND FOOD CHEMISTRY, Bd. 40, Nr. 9, 1992, Seiten 1496-1500, XP000306767 * Seite 1498, rechte Spalte; Abbildung 3 * --- | 1-7 | **RECHERCHIERTE SACHGEBIETE (Int.Cl.7)** A61K A61P |
| Y | WO 00 39248 A (RAD INTERNATIONAL LTD ;REZNIK RENA (IL)) 6. Juli 2000 (2000-07-06) * Seite 4; Ansprüche 1-3 * --- | 1-7 | |

-/--

Der vorliegende Recherchenbericht wurde für alle Patentansprüche erstellt

| Recherchenort | Abschlußdatum der Recherche | Prüfer |
|---|---|---|
| MÜNCHEN | 14. Juni 2002 | Thalmair, M |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

.............................................................................
& : Mitglied der gleichen Patentfamilie,übereinstimmendes Dokument

EPO FORM 1503 03.82 (P04C03)

Europäisches
Patentamt

**EUROPÄISCHER RECHERCHENBERICHT**

Nummer der Anmeldung

EP 02 00 2526

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int.Cl.7) |
|---|---|---|---|
| Y | DATABASE WPI<br>Section Ch, Week 200035<br>Derwent Publications Ltd., London, GB;<br>Class B04, AN 2000-402930<br>XP002202227<br>& JP 2000 119156 A (KOSE KK),<br>25. April 2000 (2000-04-25)<br>* Zusammenfassung *<br>--- | 1-7 | |
| Y | DATABASE WPI<br>Section Ch, Week 199709<br>Derwent Publications Ltd., London, GB;<br>Class B04, AN 1997-095416<br>XP002202228<br>& JP 08 333267 A (MARUZEN SEIYAKU KK),<br>17. Dezember 1996 (1996-12-17)<br>* Zusammenfassung *<br>--- | 1-7 | |
| Y | DATABASE WPI<br>Section Ch, Week 199612<br>Derwent Publications Ltd., London, GB;<br>Class B04, AN 1996-112626<br>XP002202229<br>& JP 08 012565 A (SHISEIDO CO LTD),<br>16. Januar 1996 (1996-01-16)<br>* Zusammenfassung *<br>----- | 1-7 | RECHERCHIERTE SACHGEBIETE (Int.Cl.7) |

Der vorliegende Recherchenbericht wurde für alle Patentansprüche erstellt

| Recherchenort | Abschlußdatum der Recherche | Prüfer |
|---|---|---|
| MÜNCHEN | 14. Juni 2002 | Thalmair, M |

**EP 1 314 435 A1**

## ANHANG ZUM EUROPÄISCHEN RECHERCHENBERICHT
## ÜBER DIE EUROPÄISCHE PATENTANMELDUNG NR.

EP 02 00 2526

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

14-06-2002

| Im Recherchenbericht angeführtes Patentdokument | | Datum der Veröffentlichung | Mitglied(er) der Patentfamilie | | Datum der Veröffentlichung |
|---|---|---|---|---|---|
| WO 0039248 | A | 06-07-2000 | AU | 1678700 A | 31-07-2000 |
| | | | WO | 0039248 A1 | 06-07-2000 |
| | | | AU | 1678600 A | 31-07-2000 |
| | | | EP | 1156995 A1 | 28-11-2001 |
| | | | WO | 0039066 A1 | 06-07-2000 |
| | | | US | 6383543 B1 | 07-05-2002 |
| JP 2000119156 | A | 25-04-2000 | KEINE | | |
| JP 8333267 | A | 17-12-1996 | KEINE | | |
| JP 8012565 | A | 16-01-1996 | KEINE | | |

EPO FORM P0461

Für nähere Einzelheiten zu diesem Anhang : siehe Amtsblatt des Europäischen Patentamts, Nr.12/82

8